# EUROPEAN PATENT APPLICATION

(11) **EP 2 653 860 A1**
(43) Date of publication of application: **23.10.2013**
(21) Application number: 13176540.6
(22) Date of filing: 07.12.2011
(51) Int. Cl.: G01N 27/22, A01G 25/16

(54) **An irrigation apparatus and a sensor therefor**

(30) Priority: 13.12.2010 GB 201021033
(62) Divisional of application: 11813884.1
(71) Applicant: Verdirrigation Ltd, Hull HU2 0PP (GB)
(72) Inventor: Lister, Gary, Hull, HU2 0PP (GB); Street, Alvey, Bolsover, S44 6TX (GB); Cordingley, Chris, Huddersfield, HD8 9TH (GB); Shepherd, Ray, 8600-125 Luz Lagos (PT)
(74) Representative: Bates, Daniel Joseph

(57) **Abstract**

The invention relates to a moisture sensor 16 for an irrigation apparatus 10, 100, 118, 121, 130, 140. The moisture sensor 16 being operable to monitor a moisture content for a medium in which it is located to provide a control signal for operating the irrigation apparatus 10, 100, 118, 121, 130, 140 for controlling a water supply 26 to the medium. The moisture sensor 16 comprises a sensing capacitor 46.

## Description

### Technical Field

The present invention relates to an irrigation apparatus and a sensor therefor.

### Background

Irrigation is usually required to grow crops and other plants in warmer climates where there may be insufficient water. Irrigation may also be required where improved growth is desired, or where additional water is needed to grow a particular crop or plant. Such a crop or plant may need to be grown in soil having relatively precise moisture content to obtain the required growth.

It is generally an aim in warmer climates to conserve water resources where possible because water that is suitable for irrigation may be scarce. In the future it is thought that insufficient water for irrigation will be a major factor in the ability to grow enough crops to feed the human population of the world. In such warmer climates it is frequently the case that water companies struggle to supply the demand for water with the result that water supplies may be turned off for periods of time. The consequence of stopping water supplies is that plants or crops may suffer or even die, and the result may be a failed or an inferior crop.

It is known to provide a spray or sprinkler irrigation apparatus for use in large gardens and general horticultural applications such as growing crops in fields or glasshouses. A shower or spray of water is provided in the air which falls onto the plants and ground in an area to be irrigated. A problem associated with such spray or sprinkler irrigation systems is that the water is mostly lost due to evaporation, for example, when it lands on hot ground around the plant or crop, which may be particularly the case in warmer climates. Furthermore, when there are windy conditions the water spray may be blown away from the area to be watered.

Shower or spray irrigation systems also have the problem that the water is delivered at the ground surface which is of greater benefit to weeds rather than to the plants or crops which are the target of the irrigation. The plants or crops may be deprived of water because the weeds may grow more quickly which represents a further waste of water. Most plant diseases and pests, together with their associated eggs or larvae, are also at the ground surface which is near to the plants or crops that are being watered. Delivering the water onto the ground surface may further increase the potential for plant diseases and pests. In spite of these problems shower or spray irrigation systems do offer some advantages, and are widely used.

It is further known to provide a subsoil irrigation system in glasshouses which comprises a pipe buried below the ground surface. Water having nutrients is delivered to the plants from below, which is absorbed by roots of the plants. Excess water and nutrients may run off the irrigation area and be collected for recycling in the subsoil irrigation system. In general the subsoil irrigation system requires pipes with apertures for delivery of the water, and valves to turn the water supply on and off. With such a subsoil irrigation system it is difficult to determine when the plants have received too much or insufficient water which may create the problem of over watering or under watering.

It is also known to use a sensor with an irrigation apparatus to determine a moisture content of the soil prior to watering. Typically the sensor comprises two probes which are placed in the soil and which are operable to measure an electrical resistance of the soil due to the presence of moisture. Such a sensor may not provide an accurate reading for the moisture content, and the overall arrangement of the sensor within the irrigation apparatus may be susceptible to errors which may cause over watering or under watering.

It is broadly an object of the present invention to address one or more of the above mentioned disadvantages of the previously known irrigation techniques.

### Summary

What is required is a way of irrigating, which may reduce or minimise at least some of the above-mentioned problems.

According an aspect of the invention there is provided a moisture sensor for an irrigation apparatus, the moisture sensor being operable to monitor a moisture content for a medium in which it is located to provide a control signal for operating the irrigation apparatus for controlling a water supply to the medium, wherein the moisture sensor comprises a sensing capacitor.

Such a moisture sensor has the advantage of causing an electric field of the sensing capacitor to pass through the medium in which the sensing capacitor is located. Such a moisture sensor provides the advantage of being able to control the amount of water that is used for irrigation, which may have the advantage of avoiding or reducing water from being wasted. Such a moisture sensor may also help to reduce over watering or under watering. In general a more accurate reading for the moisture content of the soil may be achieved which may permit the required amount of water for irrigation to be delivered. This may have the advantage of optimising growth for a particular crop or plant.

Preferably the sensing capacitor has at least two electrodes, wherein at least a portion of each electrode is arranged to lie in a substantially common plane. Such an arrangement has the advantage of causing electric field of the sensing capacitor to exit and enter the common plane such that it passes through a larger amount of the medium in which the sensing capacitor is located.

Preferably each said portion of the electrodes is elongate having a respective axis, and said portions are arranged such that each axis lies on a common axis. This may have the advantage of causing the electric field to pass through a larger amount of the medium in which the sensing capacitor is located.

Preferably said portions of the electrodes are substantially planar.

In one embodiment each face of said portion has a surface area of between 300 - 600 mm². Preferably each face of said portion has a surface area of substantially 450 mm². Such dimensions are a convenient size to obtain moisture readings when the moisture sensor is located in soil.

In one embodiment at least one of the electrodes has a thickness of between 20 - 50µm. Preferably at least one of the electrodes has a thickness of substantially 35µm. The use of such thin electrodes may further cause the electric field to pass through the medium in which the sensing capacitor is located.

In one embodiment the electrodes are provided by a metal etched layer of a Printed Circuit Board. Such an arrangement has the advantage of providing the electrodes in the same plane and to a degree of accuracy that is a readily repeatable during manufacturing of the moisture sensor.

Preferably the moisture sensor comprises an oscillator circuit coupled to the sensing capacitor. Preferably the moisture sensor is configured as a unitary item comprising an oscillator circuit coupled to the sensing capacitor. Such a configuration may further assist with improving the accuracy of moisture reading taken using the moisture sensor.

Preferably the oscillator circuit comprises an operational amplifier. Such an operation amplifier is a stable device which may further assist with improving the accuracy of moisture reading taken using the moisture sensor.

Preferably the operational amplifier is operable to charge and discharge the sensing capacitor to provide an output signal from the oscillator circuit having a frequency being dependent on a moisture content of a medium in which the moisture sensor is located. The provision of a frequency dependent output may provide a consistent and accurate way to measure moisture content.

Preferably a microcontroller is operable to receive the output signal to determine the frequency thereof. Using a microcontroller may further improve the accuracy of measurement of the moisture content.

In another embodiment the oscillator circuit comprises a first semiconductor switch. Preferably the sensing capacitor is coupled to a modulating capacitor which is chargeable via the first semiconductor switch. Preferably the oscillator circuit comprises a second semiconductor switch which is operable to alternatively couple the sensing capacitor to a resistor and the modulating capacitor. Preferably the modulating capacitor is coupled to the operational amplifier, such that when a high voltage threshold of the modulating capacitor is reached the operational amplifier has a low voltage output, and when a low voltage threshold of the modulating capacitor is reached the operational amplifier has a high voltage output. Such an arrangement of the oscillator circuit may further improve the accuracy of measurement of the moisture content.

Preferably the operational amplifier is coupled to a pulse counter which is operable to register a count according to the voltage output from the operational amplifier. Preferably the pulse counter comprises a microcontroller which is operable to determine the frequency of counts. Preferably the microcontroller is programmable to determine the number of counts over a preset time period to determine the frequency of counts, the preset time period being adjustable. Preferably the microcontroller has a programmable memory to calibrate the oscillator circuit and/or the sensing capacitor. Using such a microcontroller may further improve the accuracy of measurement of the moisture content.

Preferably a power source for the oscillator circuit comprises a voltage regulator. Such a voltage regulator may provide a more stable power source which may improve the operation of the oscillator circuit leading to a more accurate determination of the moisture content.

Preferably the moisture sensor further includes a buffer amplifier. Such an arrangement may improve the signal from the moisture sensor.

According to another aspect of the invention there is provided an irrigation apparatus including a moisture sensor according to the first aspect of the invention.

According to another aspect of the invention there is provided a method of using a moisture sensor in an irrigation apparatus, the moisture sensor comprising a sensing capacitor, the method including using the moisture sensor to measure a moisture content for a medium in which the moisture sensor is located to provide a control signal, and operating a water supply to the medium in response to the control signal.

Preferably the moisture sensor comprises an oscillator circuit coupled to the sensing capacitor, and the oscillator circuit comprises an operational amplifier, the method including charging and discharging the sensing capacitor using the operational amplifier to provide an output signal from the oscillator circuit that has a frequency which is dependent on a moisture content of the medium.

According to another aspect of the invention there is provided a moisture sensor according to the first aspect of the invention for use with an irrigation apparatus of the other aspects of the invention.

### Brief Description of the Drawings

Other features of the invention will be apparent from the following description of preferred embodiments shown by way of example only with reference to the accompanying drawings, in which;
**Figure 1** shows an irrigation apparatus, according to an embodiment of the invention;
**Figure 2** shows a circuit diagram for a moisture sensor used with the irrigation apparatus of Figure 1;
**Figure 3** shows a planar cross section of the moisture sensor shown in Figure 1;
**Figure 4** shows a side view cross section of the arrangement shown in Figure 3;
**Figure 5** shows a schematic diagram illustrating an electric field of the moisture sensor shown in Figures 1, 3 and 4;
**Figure 11** shows a circuit diagram for a moisture sensor according to another embodiment for use with the irrigation apparatus;
**Figure 12** shows a circuit diagram for a moisture sensor according to another embodiment for use with the irrigation apparatus; and
**Figure 13** shows a schematic diagram for the moisture sensor and control device according to an embodiment for use with the irrigation apparatus.

### Detailed Description

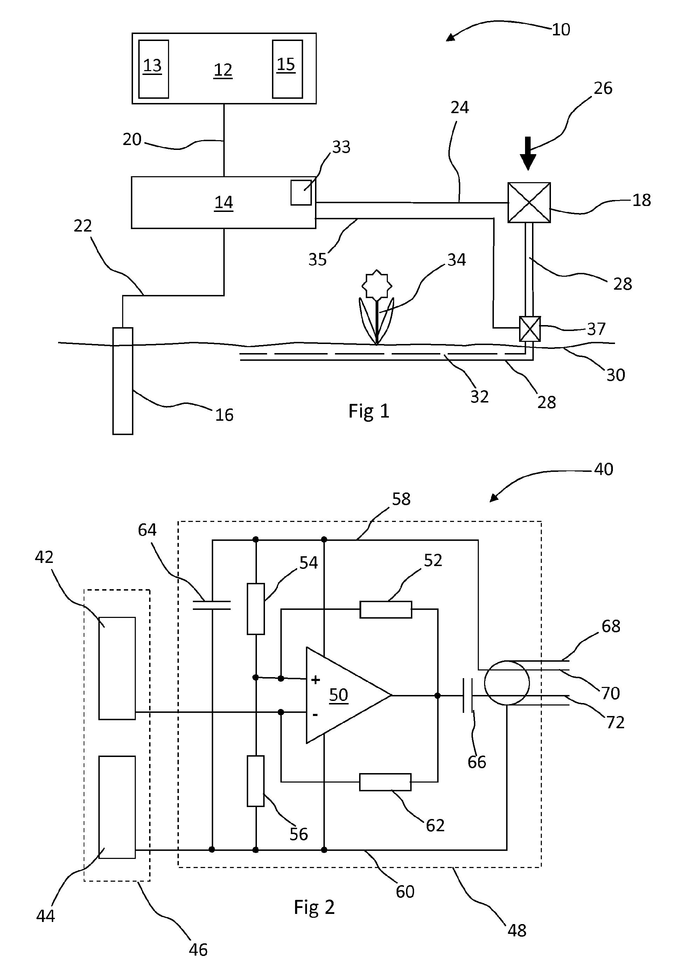

Figure 1 shows an irrigation apparatus according to an embodiment of the invention, generally designated 10. The irrigation apparatus 10 comprises a master controller 12, a control device 14, a moisture sensor 16, and a control valve 18. The master controller 12 is in communication with the control device 14 via a twin core wire 20. The control device 14 is in communication with the moisture sensor 16 via a twin core wire 22. The control device 14 is in communication with the control valve 18 via a twin core wire 24. The control valve 18 may alternatively be termed a valve device. The master controller 12 has a power supply 13. The irrigation apparatus 10 operates with a low voltage power supply 13 and current, and does not consume a large amount of power. This is advantageous because the irrigation apparatus 10 may be operational for long periods of time. The twin core wires 20, 22, 24 provide electrical power from the power supply 13 to operate the control device 14, the moisture sensor 16, and the control valve 18. In addition, the twin core wires 20, 22, 24 provide a way of communicating between the master controller 12, the control device 14, the moisture sensor 16, and the control valve 18 via a signal which is superimposed over the twin core wires 20, 22, 24. The signal may be termed a system bus which may use an appropriate communications protocol such as a 1-wire protocol. The 1-wire protocol is a known technology which is a serial protocol having a single data line and a ground reference for communication purposes.

Such a 1-wire technology requires no separate power supply for operation and takes its power from the power supply 13 used to power the master controller 12, the control device 14, the moisture sensor 16 and the control valve 18. Accordingly the 1-wire technology operates via a parasitic power supply. It will be appreciated that the 1-wire technology is a voltage-based digital system that works with two wires for half-duplex bidirectional communication.

In an alternative embodiment the wires 20, 22, 24 are replaced by wireless communication links, and the power for operating the components of the irrigation apparatus 10 may be provided locally to each of the control device 14, the moisture sensor 16 and the control valve 18, for example by using a battery in each component. The control device 14 may be provided as a portable device which is operable to be moved in the vicinity of each moisture sensor 16 so that a moisture content reading can be captured. In such an arrangement the system bus is provided by a wireless communications protocol to allow such moisture content readings to be captured. A suitable wireless communications protocol might be a Bluetooth protocol which has a relatively short range of operation. Other types of wireless communication links may be used which operate by using an electric field, or a magnetic field, or a combination of the electric and magnetic fields.

In either embodiment the control device 14, the moisture sensor 16, and the control valve 18 may have unique identification addresses, such as Internet Protocol (IP) addresses, for identification and addressing purposes using the system bus. Such addresses permit instructions to be sent to specific components of the irrigation apparatus 10. The unique addresses may be assigned to the various components of the irrigation apparatus 10 when it is used for the first time. The master controller 12 is also operable to change the addresses as required.

The control valve 18 is connected to a water supply 26 and a delivery pipe 28 which can be buried below a ground surface 30. The water supply 26 is a supply of water to the irrigation apparatus 10, which is under pressure. The delivery pipe 28 may be alternatively termed a delivery duct. The control valve 18 is operable to allow water to pass from the water supply 26 to the delivery pipe 28 on command. The delivery pipe 28 has at least one aperture 32 in a portion of the delivery pipe 28 which is below the ground surface 30 for watering a plurality of plants 34. In an alternative arrangement only the control device 14 and the control valve 18 are provided with an IP address. In an alternative arrangement only the control device 14 is provided with an IP address. Such arrangements may simplify the data collection activities for the master controller 12. In an alternative embodiment the portion of the delivery pipe 28 with the aperture 32 may by above the ground surface 30 so that the irrigation apparatus operate as a drip irrigation system. In an alternative arrangement the delivery pipe 28 may be used to provide water to a sprinkler or spray irrigation system. It will, however, be appreciated that delivering the water underground in the vicinity of plant roots may further reduce water consumption and may help to reduce pests and diseases.

The moisture sensors 16 may be manufactured in batches. After manufacture each batch of moisture sensors 16 is tested for sensitivity, and to ensure that they are manufactured within specification. This may be achieved by taking a sample of each batch and testing them when suspended in air to ensure that they provide the correct dry reading. When testing the moisture sensors 16 in air no other substance should be within 50mm of the two flat faces to avoid distortion of the dry reading. The moisture sensors 16 are then tested with a 50mm thick polythene block touching each flat face of the moisture sensor 16 to ensure that they provide the correct wet reading. Such a polythene block may be more convenient than immersing the moisture sensors 16 in water to obtain the wet reading. If the dry and wet readings are not within specification, the whole batch can be adjusted by changing the value of a component within the moisture sensors 16. Since this change is the same for each moisture sensor 16 within the batch it is a straight forward task to complete for the whole batch. It will be appreciated that the above way of testing the batches of moisture sensors 16 is completed in a factory environment and may be carried out in an automated manner. Since all moisture sensors 16 can be tested at the factory this provides the advantage of allowing moisture sensors 16 to be added or removed from the irrigation apparatus 10 more easily.

In use, when the moisture sensor 16 is placed under the ground surface 30, the control device 14 is operable to determine a moisture reading for the soil around the moisture sensor 16. The master controller 12 may then interrogate the control device 14 so that it has knowledge about the moisture content below the ground surface 30. In operation the master controller 12 is programmable with a desired moisture content level, also known as a predetermined threshold value, for a particular area containing the plants 34. The moisture sensor 16 and the control device 14 continuously monitor the moisture content level below the ground surface 30. If the moisture content measured by the moisture sensor 16 falls below the desired moisture content the control device 14 initiates operation of the control valve 18 so that water passes into the delivery pipe 28 from the supply water 26 to irrigate the area around the plants 34. In an alternative embodiment the moisture sensor 16 and the control device 14 periodically monitor the moisture content below the ground surface 30. Such an arrangement may help to reduce the amount of power consumed by the irrigation apparatus 10. Such an arrangement assists with providing the correct amount of water to the plant 34 so that over watering or under watering is avoided or reduced. This has the advantage of reducing the possibility that the water is wasted.

A memory 33 may be provided at the control device 14 for storing the moisture readings of the soil in which the moisture sensor 16 is located. In an alternative embodiment the memory 33 is located at the moisture sensor 16 instead of the control device 14. The master controller 12 may be arranged to interrogate the memory 33 to determine the moisture readings. Such alternative arrangements may provide advantages when replacing certain parts of the irrigation apparatus 10.

It will be appreciated that when operating the irrigation apparatus 10 in a large area, such as a field where there are many plants 34, the irrigation apparatus 10 may have many control devices 14 for controlling the moisture content of the soil below the ground surface 30. In a preferred arrangement each control device 14 is in communication with only one moisture sensor 16. Furthermore, there may be many control devices 14 in communication with the master controller 12. It is envisaged that there may be up to 240 or more moisture sensors 16 which are in communication with respective control devices 14, whereby each control device 14 is in communication with a single master controller 12. In addition, there may be many control valves 18 and delivery pipes 28 to irrigate many different areas containing the plants 34. The control valves 18 may be operated by one or more control devices 14 as required. In an alternative arrangement one control device 14 may operate many control valves 18, for example up to ten control valves 18, where the control valves 18 are operated to open or close substantially at the same time. With such an arrangement the control device 14 may be relatively local to the moisture sensors 16, and the master controller 12 may be located relatively distant from the control device 14. It is envisaged that the moisture sensors 16 may be up to 100m away from the control device 14, and the master controller may be up to 200m away from the control device 14. Each control device 14 may be a member of a particular group in an area to be watered. It is envisaged that there may be 12 groups with each group having 20 control devices 14. The master controller 12 is operable to read a moisture value from a moisture sensor 16 attached to a particular control device 14, and to write the value to a database 15 containing a list of addresses of the moisture sensors 16. The database 15 is typically within the master controller 12.

The provision of the system bus may allow the control devices 14, the moisture sensors 16, and the control valves 18 to be readily added or removed from the irrigation apparatus 10 as required which is useful when using the irrigation apparatus 10 in a large area such as a field. In one embodiment, one moisture sensor 16 is in a middle region of a certain area to be irrigated, such as a flower bed containing plants 34, and the control device 14 is operable to send an activation command to one control valve 18 so that all plants 34 in the flower bed are provided with the required water.

In one embodiment the master controller 12 is a computer-based device which has a processor, a display apparatus for displaying information, a memory, a keyboard for inputting data, indicator lights, and one or more audiovisual alarms. The memory is operable to record moisture readings from moisture sensors 16. The processor is operable to issue commands to the control devices 14 to operate the control valves 18. The control devices 14 may also have a processor to receive commands and execute them. If the master controller 12 determines that a particular moisture content reading from a particular moisture sensor 16 is below the threshold value it sends a command signal to the control device 14 to activate the control valve 18 so that water is supplied to the delivery pipe 28. Accordingly, the irrigation system 10 is a feedback system which continuously or periodically monitors moisture in the soil below the ground surface 30, and activates the control valve 18 to supply water to the soil if the moisture content falls below the threshold value for the moisture content reading. Using such an arrangement permits the master controller 12 to read, report, control, and modify settings and thresholds on all components of the irrigation apparatus 10, which may be a useful feature to adjust the amount of water used due to seasonal and climatic variations.

The master controller 12 is programmable with the time and date which may be useful when setting the watering times for the area to be irrigated according to the season. For example, longer watering times would typically be required in summer than in winter. Typically the master controller 12 is programmable with one or more watering cycles having a start time and a finish time. This information can be used to determine a length of time for each watering cycle. The length of time for watering may be combined with a known flow rate for the water supply 26 so that the volume of water used for irrigation can be calculated.

The master controller 12 permits the component parts of the irrigation system 10 to be operated from one central location. Once the irrigation apparatus 10 has been installed there is no need for user contact with the moisture sensors 16 or the control device 14. All data from the moisture sensors 16, including data from the control valves 18 which may relate to water consumption is available at the master controller 12. Furthermore, the master controller 12 is capable of calculating drying out rates of the various soil areas, and the amount of water that has been saved using the irrigation apparatus 12. In one embodiment the master controller 12 has a port, such as a USB port, for connecting it to a computer, such as a Personal Computer (PC), via the RS232 standard, the Controller-Area Network (CAN) bus standard, or the Ethernet protocol, or Wireless protocol, which may allow a computer application to supervise and/or operate the master controller 12. The PC may be remote from the master controller 12 and is capable of programming it, and of running software to receive data and error reports relating to the overall operation of the irrigation apparatus 10. The port also allows data for the irrigation apparatus 10 to be downloaded and analysed as required. In the case of a wireless connection between the PC and the master controller 12, the PC may be located in a vehicle so that it can interrogate and program a plurality of the master controllers 12 in turn as it passes by.

The control device 14 is capable of operating any type of control valve 18 that is suitable for controlling the supply of water 26. Accordingly, the control valve 18 may be an electrically operated valve or any other type of valve such as a pneumatic, hydraulic or gear driven control valve 18. The control valve 18 may be a bi-stable valve which is opened by a first signal and remains open until it is closed by a second signal. Alternatively the control valve 18 may remain open as long as power is applied to it. The bi-stable arrangement may consume less electrical power and may allow a greater number of control valves 18 to be open at the same time. It will be appreciated that the control device 14 may be programmable with software to provide the various alternative functions, and that this software may be loaded onto the control device 14 from the master controller 12 via the system bus. Accordingly, the loading of different software onto the control device 14 does not require it to be physically disconnected from the irrigation apparatus 10.

In one embodiment one moisture sensor 16 is in communication with one control device 14, and the one control device 14 is in communication with many control valves 18, such as up to three control valves 18. Optionally the control device 14 may also be in communication with the master controller 12. With this arrangement all of the control valves 18 in communication with the control device 14 operate together, and at substantially the same time. The identification address may be associated with the control device 14 only, so that if the master controller 12 is required to activate a valve device 18 it sends a message to the control device 14 associated with the one or more control valves 18. The message is a signal to the control device 14 to operate the one or more control devices 18 in communication with the control device 14.

In another embodiment the irrigation apparatus 10 does not have a master controller 12, and the decision on whether to water or not is made by the control device 14. This is achieved by programming the control device 14 with appropriate software to perform the decision making function. In this arrangement the control device 14 is provided with its own power supply for operating it and the control valve 18. In this arrangement a processor of the control device 14 performs the management and control functions of the irrigation apparatus 10.

Figure 2 shows a circuit diagram, generally designated 40, for the moisture sensor 16 used with the irrigation apparatus 10 of Figure 1. In Figure 2, the circuit diagram 40 shows a first and a second electrode 42, 44 which form a sensing capacitor 46 having a capacitance centred around 10pF. The remainder of the circuit 40 comprises an oscillator circuit 48 to drive the sensing capacitor 46. In use, the dielectric for the sensing capacitor 46 comprises the medium in which the moisture sensor 16 is located, such as soil or air. The capacitance of the sensing capacitor 46 provided by the electrodes 42, 44 is dependent on the permitivity of the dielectric material between or surrounding the plates. Water has a dielectric constant or permitivity of about 80 and dry air has a permitivity of 1.

The oscillator circuit 48 comprises an operational amplifier 50 configured with two feedback paths between an output from the operational amplifier 50 and two inputs to the operational amplifier 50. A first feedback path is via a 1MΩ resistor 52 to the non-inverting input of the operational amplifier 50. The non-inverting input is also connected to the centre tap of a pair of 1MΩ resistors 54, 56 that are connected in series between a positive supply wire 58 and a negative supply wire 60 forming a partial circuit. This partial circuit forms a Schmitt trigger creating a square wave at the output of the operational amplifier 50. The feedback proportion is limited so that a peak to peak voltage differential across the two inputs to the operational amplifier 50 is about 1.66 Volts when using a 5 Volt power supply. Such an arrangement ensures that the input protection diodes of the operational amplifier 50 do not conduct, and the input impedance of the operational amplifier 50 remains typically above 45MΩ. A second feedback path of the oscillator circuit 48 is from the output of the operational amplifier 50 to the inverting input of the operational amplifier 50 via a 1MΩ resistor 62 and the sensing capacitor 46. The sensing capacitor 46 is between the inverting input of the operation amplifier 50 and the negative power supply wire 60.

The oscillator circuit 48 works in the following manner. When power is first applied to the oscillator circuit 48 the voltage on the non-inverting input of the operational amplifier 50 approaches half of the voltage of the power supply wires 58, 60 more quickly than the voltage of the inverting input of the operational amplifier 50. The latter voltage is slowed by the presence of the sensing capacitor 46 between the power supply wire 60 and the non-inverting input of the operational amplifier 50. A capacitor 64 is also provided between the power supply wires 58, 60, which has a value of 100nF. The capacitor 64 operates to remove or reduce any noise that may be present on the power supply wires 58, 60 which provides a more stable output from the oscillator circuit 48. At this time the output voltage from the operation amplifier 50 will be close to its positive supply voltage. The voltage of the sensing capacitor 46 will become more positive as it receives charge from the output of the operational amplifier 50 via the resistor 62. After approximately 10 µs the voltage of the sensing capacitor 46 will be more positive than the voltage of the non-inverting input of the operational amplifier 50 which causes the output of the operational amplifier 50 to become less positive. This in turn reduces the voltage on the non-inverting input which reinforces the change in output voltage of the operational amplifier 50 until it is very close to the negative power supply wire 60. The charge on the sensing capacitor 46 is then reduced by current flowing from it through the resistor 62 to the output of the operational amplifier 50. After a further period of about 10 µs the voltage of the sensing capacitor 46 falls by about 1.6V so that it is the same as the voltage of the non-inverting input of the operational amplifier 50. The output voltage of the operational amplifier 50 then changes quickly until it approaches the voltage of the positive supply wire 58 and the whole process repeats. Overall a square wave is produced at the output of the operational amplifier 50 which has a variable frequency centred around approximately 50 KHz. The output of the operational amplifier 50 is also provided with an output capacitor 66 having a value 0.1µF. The output capacitor 66 acts as a DC blocking capacitor that provides some protection to the operational amplifier 50 in the event of the output being inadvertently short circuited to the power supply wires 58, 60. The output capacitor 66 may be replaced by a resistor having a value of 10kΩ, which provides the same kind of protection at a lower cost.

Also shown in Figure 2 are three wires 68, 70, 72 which are arranged as a pair of wires 70, 72 surrounded by the third wire 68 which forms an electrical screen. The wire 70 supplies electrical power to the oscillator circuit 48, and the other wire 72 is the output signal from the oscillator circuit 48. The electrical screen formed by the wire 68 also forms the return path for the DC electrical power supply and for the square wave output signal from the oscillator circuit 48.

It will be appreciated that the operation of the oscillator circuit 48 is dependent on charging and discharging of the sensing capacitor 46 through the resistor 62. This charging and discharging is terminated when the voltage between the electrodes 42, 44 of the sensing capacitor 46 reaches a specific voltage. The charging and discharging rate is also dependent upon the same voltage. If the supply voltage to the electrodes 42, 44 increases, the rate of charging of the sensing capacitor 46 increases, but at the same time the voltage changes which reduces the rate of charging of the sensing capacitor 46. One effect cancels out the other, so that the time to reach the switching point is independent of the supply voltage to the sensing capacitor 46. The frequency of oscillation of the voltage of the sensing capacitor 46 depends only on the capacitance of the sensing capacitor 46, and the value of the resistor 62. The variance of the output frequency of the oscillator with climatic temperature of the capacitance of the sensing capacitor 46 and the resistance of the resistor 62 is less than 100 parts per million per °C as discussed in more detail below. Accordingly, the sensing capacitor 46 and the oscillator circuit 48 are highly tolerant to climatic temperature changes. Furthermore, the variance with climatic pressure of the capacitance of the sensing capacitor 46 and the resistance of the resistor 62 is negligible.

The accuracy of the moisture reading provided by the moisture sensor 16 of the soil below the ground surface 30 is dependent on the repeatability and accuracy of the oscillator circuit 48. Since the oscillator circuit 48 is highly tolerant to climatic temperature and pressure changes due to its design, each moisture sensor 16 in the irrigation apparatus 10 provides a very similar moisture reading to other moisture sensors 16 in the irrigation apparatus 10. Moisture sensors of the prior art have used a microprocessor with an internal oscillator within the microprocessor, but these oscillators are not controlled, are unstable, and require adjustment in the production process.

In normal use the oscillator circuit 48 of an embodiment of the invention will operate with a voltage provided by the power supply wires 58, 60 in the range 3.3V to 5 V. However, the oscillator may also operate with a voltage as low as 1.8V or as high as 5.5V. The current draw of the oscillator circuit 48 is variable with temperature, but this is designed to be less than 1mA. The total power consumption of the oscillator circuit 48 is typically less than 2mW.

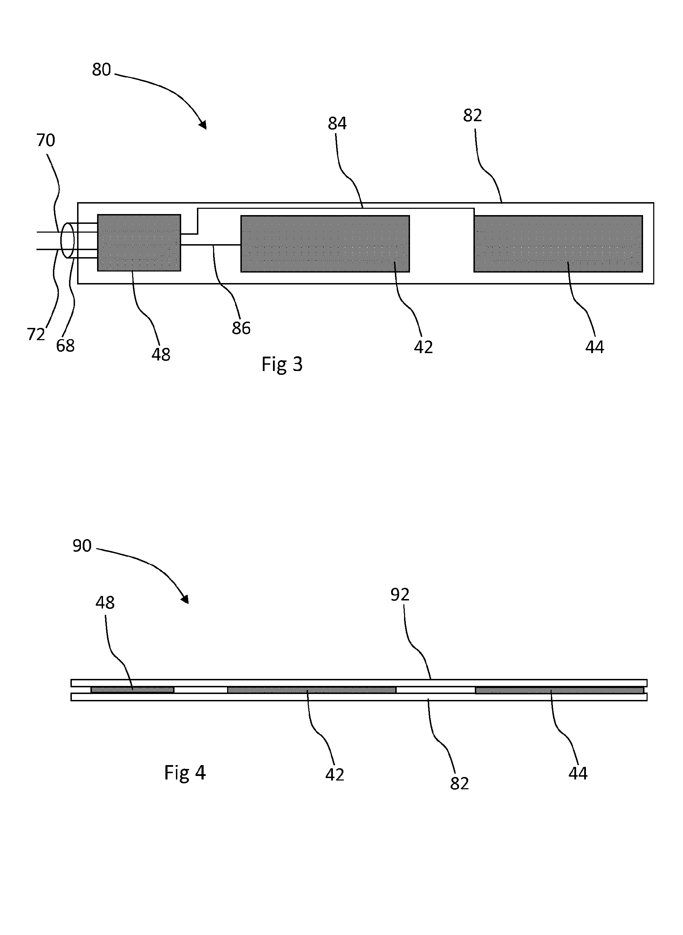

Figure 3 shows a planar cross section of the moisture sensor shown in Figure 1, generally designated 80. In Figure 3 like features to the arrangements of Figure 2 are shown with the same reference numerals. In Figure 3 the oscillator circuit 48, and the two electrodes 42, 44 are shown to be mounted on a first Printed Circuit Board (PCB) layer 82 which may be a woven glass and epoxy material such as FR-4 which is 1.6mm thick. The first PCB layer 82 has a layer of copper on its surface which is 35µm thick with the two electrodes 42, 44 being etched from the copper surface. Accordingly the two electrodes 42, 44 are substantially flat and arranged to lie in a single plane. The two electrodes 42, 44 are substantially the same size. The two electrodes 42, 44 are connected to the oscillator circuit 48 by copper tracks 84, 86 that are also etched onto the PCB. This arrangement provides a robust and reliable moisture sensor 16. Such an arrangement is also relatively simple to manufacture which helps to reduce manufacturing costs. Each electrode 42, 44 has the same area on the first PCB layer 82 of about 50mm X 10mm.

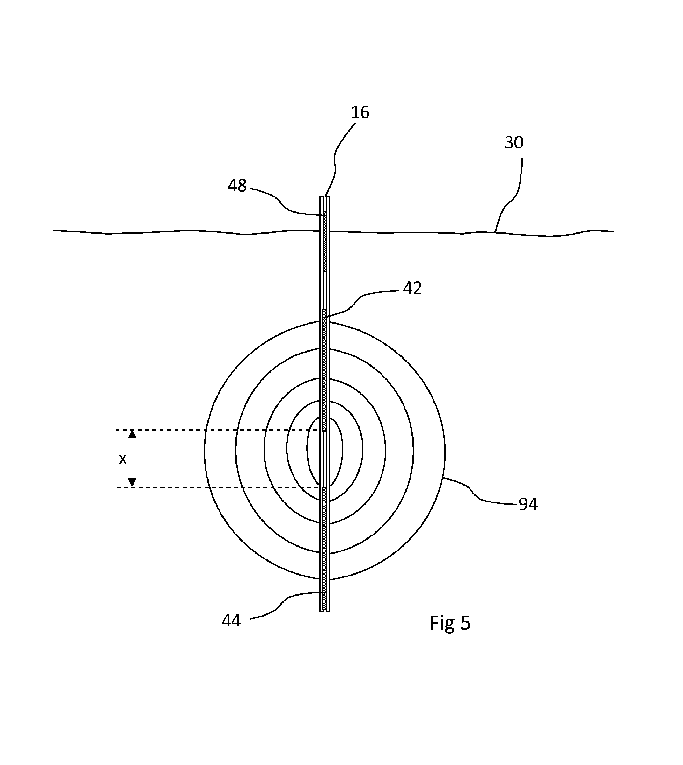

Figure 4 shows a side view cross section of the arrangement shown in Figure 3, generally designated 90. In Figure 4 like features to the arrangements of Figure 3 are shown with like reference numerals. In Figure 4 a second PCB layer 92 is shown to be bonded on top of the first PCB layer 82 so that the moisture sensor 16 comprises a sandwich of the two layers of PCB material 82, 92. The second PCB layer 92 does not have a copper coating. The bonding of the two layers of PCB material 82, 92 may be provided by an Epoxy resin so that the moisture sensor 16 is a waterproof assembly where no electrical contact is made with the soil or other medium into which the probe is located. The assembly may also be encased in a plastic material. Each electrode 42, 44 is elongate having an axis along the moisture sensor 16, and the two electrodes 42, 44 are arranged to lie end-on to each other so that each axis lies on a common axis. Figure 4 also shows that the electrodes 42, 44 are relative thin. Such thin electrodes 42, 44 combined with their relatively large area is useful because it has the effect of forcing an electric field of the sensing capacitor 46 out of the plane in which they are located as shown at 94 in Figure 5. In particular providing relatively thin electrodes 42, 44 means that the end of each electrode 42, 44 has a relatively small area which further assists with forcing the electric field out of the surface of the electrodes 42, 44 and into the soil.

Figure 5 shows a schematic diagram illustrating the electric field 94 of the moisture sensor 16 shown in Figures 1, 3 and 4. In Figure 5 like features to the arrangements of Figure 1, 3 and 4 are shown with like reference numerals. In Figure 5 it can be seen that the electric field 94 exits and enters the electrodes 42, 44 perpendicularly to their surface. In this manner the electric field 94 passes through a relatively large volume of soil when it is under the ground surface 30 which has the effect that the capacitance between the electrodes 42, 44 is influenced by the large volume of soil which is away from the moisture sensor 16. With such an arrangement a more accurate moisture reading for the soil may be obtained using the moisture sensor 16. It will be appreciated that if the electrodes 42, 44 are further apart, such that the dimension x between the electrodes 42, 44 is increased, the electric field 94 is spread out more so that it passes through an even larger volume of soil. In Figure 5 the electrodes 42, 44 are shown to be separated by about half of their length. Accordingly the electrodes 42, 44 have a large separation relative to their area. In Figure 5 the moisture sensor 16 is shown to be vertically oriented within the soil, but it will be appreciated that it will work in any orientation as long as the electrodes 42, 44 are sufficiently below the ground surface 30 so that the electric field 94 is sufficiently below the ground surface 30.

The output frequency of the oscillator circuit 48 is a function of the capacitance of the sensing capacitor 46. The capacitance of the sensing capacitor 46 is a function of the soil moisture content. In operation the control device 14 is operable to compare the output frequency with the stored calibration information mentioned above to determine a moisture content reading for the soil. In addition, the output frequency of the oscillator circuit 48 is affected by the change in physical size of the electrodes 42, 44 due to temperature, the change in offset bias at the input to the operational amplifier 50, the temperature coefficient of the resistor 62, and the supply voltage to the oscillator circuit 48 provided by the supply wires 58, 60. Each of these effects is considered in the paragraphs below.

Regarding the change in physical size of the electrodes 42, 44 due to temperature, the change in dimension is small at about 15 parts per million per °C which has a correspondingly small effect on the output frequency of the oscillator circuit 48. The dimensions of the electrodes 42, 44 can be controlled in the manufacturing process to be within 0.05mm over 200mm, which is 250 parts per million from one batch of moisture sensors 16 to another batch of moisture sensors 16 that are manufactured. Within each batch a variation of less than 50 parts per million is typical. Accordingly, the arrangement of the moisture sensor 16 comprising the electrodes 42, 44 with the oscillator circuit 48 means that it can be manufactured so that there is a relatively small variance between the output frequencies and amplitudes of different moisture sensors 16. The variation of output amplitude and frequency is within 2% between any two moisture sensors 16. This has the advantage that each moisture sensor 16 does not require individual calibration.

Regarding the change in offset bias at the input to the operational amplifier 50 this is very small and will only influence the mark to space ratio, which is the ratio between the time the voltage is at a high or positive level, and the time that the voltage is at a low or negative level. The output frequency of the oscillator circuit 48 is not dependant on the change in offset bias.

Regarding the temperature coefficient of the resistor 62 this is typically about 25 parts per million per °C which is small. Furthermore the temperature coefficient has an initial tolerance of 0.1% over the range -40°C to +85°C. Overall this equates to better than 10 parts per million per °C.

Regarding changes in the supply voltage to the oscillator circuit 48 provided by the supply wires 58, 60, this influences the oscillator circuit 48 in two ways. One effect is to reduce the charging or discharging current for the sensing capacitor 46, and another effect is to reduce the voltage at the switching point. These both cancel each other out so there is no change in output frequency from the oscillator circuit 48.

Whereas the parameters of the operation amplifier 50 change with temperature and voltage, the output frequency of the oscillator circuit 48 is not affected. The amplitude of the output frequency may change a few percent, but the control device 14 only responds to the time between the maxima of the positive pulses and not to their amplitude when determining a moisture content reading for the soil. Furthermore, the oscillator circuit 48 being provided on the moisture sensor 16 also has the advantage of providing less variation in the output frequency from the oscillator circuit 48. Overall the construction of the moisture sensor 16 is such that it is inherently insensitive to climatic temperature or pressure changes which provides for simple manufacture and operation within the irrigation apparatus 10. The moisture sensor 16 is a unitary item comprising the oscillator circuit 48 and the sensing capacitor 46. The unitary nature of the moisture sensor 16 means that it can be readily incorporated or removed from the irrigation apparatus 10 as required. Furthermore, due to the manner of construction of the moisture sensor 16 comprising two layers of PCB material 82, 92 it has a relatively flat shape making it easy to push into soil below the ground surface 30.

If the worst case scenario occurs and all the temperature related changes influence the frequency output from the oscillator circuit 48 in the same manner, the frequency has a temperature dependence of 15 + 50 + 25 + 10 = 100 parts per million per °C. This gives a variance of 5000ppm or 0.5% over a temperature range of 0 to 50°C. In a comparison of one manufactured batch of moisture sensors 16 to another manufactured batch of moisture sensors 16 there might be a slightly worse temperature dependency of 250 parts per million per °C. Since this would be a batch problem, the whole batch could be corrected by changing the value of the resistor 62 for all moisture sensors 16 of the batch which removes the batch variance issue from the overall variance calculation.

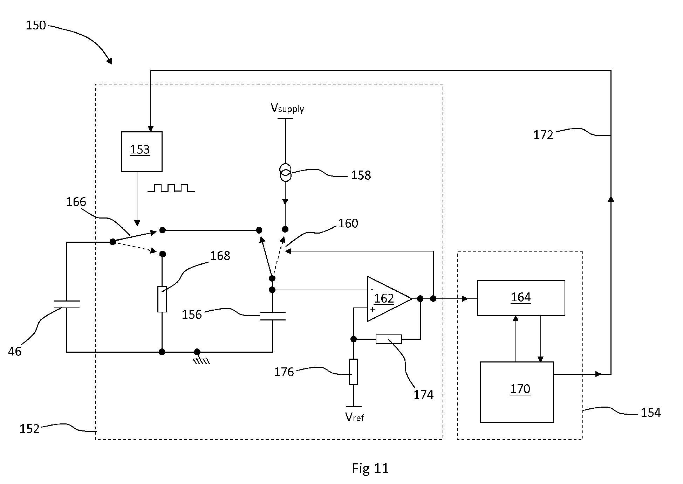

Figure 11 shows a circuit diagram for a moisture sensor according to another embodiment, generally designated 150, for use with the irrigation apparatus of the above embodiments. In Figure 11 like features to the arrangements of Figure 1 - 10 are shown with like reference numerals. In Figure 11 the circuit diagram 150 includes the sensing capacitor 46 of the moisture sensor 16 having a capacitance centred around 10pF. It will be understood that the sensing capacitor 46 has a variable capacitance which depends on the medium in which it is located. For example, the sensing capacitor 46 may be arranged to operate between 1 - 200pF, although a range between 5 - 15pF is envisaged. The remainder of the circuit 150 comprises an oscillator circuit 152, and a data processing module 154. In use, the dielectric for the sensing capacitor 46 comprises the medium in which the moisture sensor 16 is located, such as soil or air. The capacitance of the sensing capacitor 46 is dependent on the permitivity of the dielectric material between or surrounding the plates of the capacitor, which may be configured as per the first and the second electrodes 42, 44 shown in Figures 2 - 5 where they lie in a common plane. It will be appreciated that the circuit 150 shown in Figure 11 can be used with the irrigation apparatus 10, 100, 118, 120, 130, 140 of the above embodiments to control the water supply 26 to the medium in which the moisture sensor 16 is located.

In Figure 11 the sensing capacitor 46 is coupled to a modulating capacitor 156 which is charged from a constant current source 158 via a first Complementary Metal Oxide Semiconductor (CMOS) switch 160, which is a general semiconductor switch. The modulating capacitor 156 is also coupled to an operational amplifier 162, which operates as a comparator. When a high voltage threshold of the modulating capacitor 156 is reached an output of the operational amplifier 162 is low which causes a count to be registered on a pulse counter 164 of the data processing module 154. The low output of the operational amplifier 162 also causes the first CMOS switch 160 to connect the modulating capacitor 156 to a second CMOS switch 166, which is also a general semiconductor switch. An oscillator 153 causes the second CMOS switch 166 to repeatedly and alternately connect the sensing capacitor 46 between the modulating capacitor 156 and a resistor 168. This causes the sensing capacitor 46 to either charge from the modulating capacitor 156 or discharge via the resistor 168. When a low voltage threshold of the modulating capacitor 156 is reached the output of the operational amplifier 162 is high, which causes the cycle to repeat. The arrangement of the sensing capacitor 46 and the modulating capacitor 156 working together is advantageous because they operate to provide a ratio effect so that the variations of the capacitors 46, 156 caused by environmental conditions change together. This has the advantage of cancelling or filtering out the variations which leads to a more accurate measurement of the moisture level.

It will be appreciated that when the capacitance of the sensing capacitor 46 is large a smaller number of oscillation periods will be required to discharge the modulating capacitor 156, which means that the number of counts recorded by the pulse counter within a set time period will be higher. This means that the counts are at a relatively higher frequency. Furthermore, when the capacitance of the sensing capacitor 46 is small a larger number of oscillation periods will be required to discharge the modulating capacitor 156, which means that the number of counts recorded by the pulse counter within a set time period will be lower. This means that the counts are at a relatively lower frequency. The number of counts is related to the moisture content of the soil in which the sensing capacitor 46 is located, and a microcontroller 170 is coupled to the pulse counter 164 to assign a moisture content to a particular number of counts per set time period. A feedback line 172 is provided from the microcontroller 170 to the oscillator 153 for controlling the operation of the oscillator 153. The feedback line 172 is a control line which may be used to set the frequency of the oscillator 153 and to switch it on or off. The operational amplifier 162 is also provided with a feedback resistor 174 between the output and a non inverting input. A resistor 176 is also provided before the reference voltage Vref which is connected to the non inverting input.

It will be appreciated that the circuit 150 may be configured so that it fits onto the arrangement of the moisture sensor 80, 90 shown in Figures 3 and 4. In one embodiment the data processing module 154 may be provided in the control device 14 or the master controller 12 of Figures 1, 6, 7, 8, 9 or 10.

In Figure 11 the accuracy of the moisture reading provided by the sensing capacitor 46 is dependent on the repeatability and accuracy of the oscillator circuit 152. Since the oscillator circuit 152 is highly tolerant to climatic temperature and pressure changes due to its design, each moisture sensor 16 in the irrigation apparatus 10, 100, 118, 120, 130, 140 provides a very similar moisture reading to other moisture sensors 16 in the irrigation apparatus 10, 100, 118, 120, 130, 140. Furthermore, when relatively long wires are used to connect each moisture sensor 16 to the controller device 14 or the master controller 12, the circuit 150 may be less prone to electrical interference, for example due to radio waves or objects passing nearby such as trains or cars.

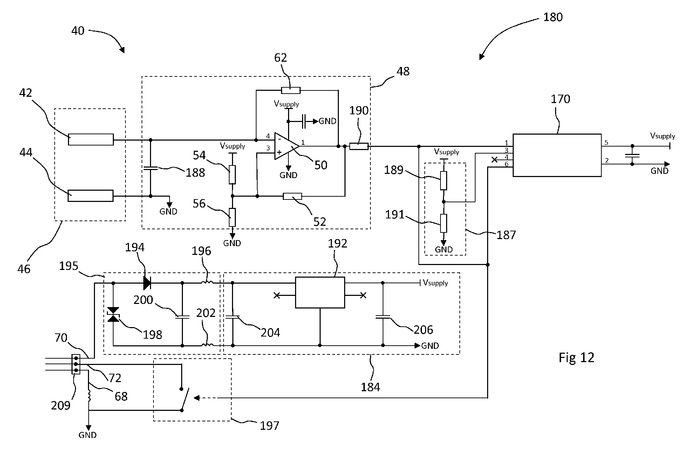

Figure 12 shows a circuit diagram for a moisture sensor according to another embodiment, generally designated 180, for use with the irrigation apparatus 10, 100, 118, 120, 130, 140 of the above embodiments. In Figure 12 like features to the arrangements of Figure 1 - 11 are shown with like reference numerals. In Figure 12 the circuit diagram 180 includes a similar arrangement to the circuit diagram 40 shown in Figure 2, and operates in a similar manner. In Figure 12 the circuit diagram 180 also includes a voltage regulator circuit 184 for driving the oscillator circuit 48 and the microcontroller 170. The microcontroller 170 may also be associated with the pulse counter 164 in a similar manner to the arrangement of Figure 11. In Figure 12 a partial circuit 187 is also shown, which may be used when the operational amplifier 50 of the oscillator circuit 48 is embedded within the microcontroller 170 itself instead of being external to it. In this arrangement the partial circuit 187 comprises two resistors 189, 191 which mimic the arrangement of the two resistors 54, 56 of the oscillator circuit 48. In Figure 12 the circuit diagram 180 also includes a protection circuit 195 and a semiconductor switch 197. It will be appreciated that the circuit 180 shown in Figure 12 can be used with the irrigation apparatus 10, 100, 118, 120, 130, 140 of the above embodiments to control the water supply 26 to the medium in which the moisture sensor 16 is located.

In Figure 12 the oscillator circuit 48 includes a capacitor 188 which is arranged in parallel with the sensing capacitor 46, and a resistor 190 in place of the capacitor 60 shown in Figure 2. In Figure 12 the oscillator circuit 48 operates in the following manner. Assuming that the sensing capacitor 46 is not charged, it initially charges through the resistor 62 from a high output of the operational amplifier 50 shown at pin 1 thereof. The voltage on the sensing capacitor 46 will rise as it charges until it exceeds the voltage of the non inverting input pin 3 of the operational amplifier 50 because it is connected to the inverting input pin 4 of the operational amplifier 50. At this point the output of the operation amplifier 50 switches to a low value. This output reduces the threshold voltage at pin 3 of the operational amplifier 50 via the resistor 52, and causes the sensing capacitor 46 to start discharging through the resistor 62. When the voltage of the sensing capacitor 46 discharges to a level below that on pin 3 of the operational amplifier 50, the output of the operation amplifier 50 switches to a high value. The cycle then repeats which provides an oscillation between charging and discharging of the sensing capacitor 46 whose frequency is dependent upon the capacitance of the sensing capacitor 46, which is dependent upon the moisture in the medium surrounding the capacitor plates.

The voltage regulator circuit 184 provides a stable power supply to the operational amplifier 50 to improve the stability of measurement of the moisture level by the capacitor 46. The voltage regulator circuit 184 operates to reduce variations in the supply voltage which may be caused by varying lengths of the three wires 68, 70, 72 which correspond to the three wires 68, 70, 72 shown in Figure 2. In Figure 12 the three wires 68, 70, 72 are attached to a connector block 209. Overall the stability of the oscillator circuit 48 is improved by the voltage regulator circuit 184. The voltage regulator circuit 184 may be configured so that it fits onto the arrangement of the moisture sensor 80, 90 shown in Figures 3 and 4, and may assist with overcoming power supply variations caused by different connecting cables and power sources.

The protection circuit 195 may be used for lightning protection, Electromagnetic Compatibility (EMC) compliance, and incorrect polarity protection. The incorrect polarity protection is provided by a diode 196. The EMC protection and lightening protection is provided by a first inductor 196, a transient suppression diode 198, a capacitor 200, and a second inductor 202.

The microcontroller 170 counts pulses at pin 1 that are output from the operational amplifier 50 over a set period of time. This period of time can be changed so that variations caused by occasional electrical interference are averaged to reduce the impact on moisture readings. Such electrical interference may be caused by, for example, radio waves or objects passing nearby such as trains or cars. The microcontroller 170 also contains a programmable memory which can be used to calibrate the oscillator circuit 48 and capacitor 46 at the manufacturing stage. Such calibration may be required due to variations in the operation amplifier 50 which may provide a variable count period. The output pin 6 of the microcontroller 170 is then provided either as a periodic signal inversely proportional to the capacitance of the sensing capacitor 46, or as a digital signal serially encoded with a value proportional to the capacitance of the sensing capacitor 46. The output signal from the microcontroller 170 at pin 6 is passed via the semiconductor switch 197 to the connector block 209 and then on to a master controller 12 or control device 14 as shown in Figures 1, 6, 7, 8, 9, 10. It is envisaged that the sensing capacitor 46 and the operational amplifier 50 may be included in the same physical device as the microcontroller 170 thereby saving space and cost. In addition the protection circuit 195, the semiconductor switch 197, and the voltage regulator circuit 184 may also be included on the same physical moisture sensor 16.

In another arrangement of the circuit diagram 180 shown in Figure 12, the oscillator circuit 152 shown in Figure 11 may be used in place of the oscillator circuit 48. Such an arrangement may provide the advantage of an improved accuracy or stability in providing a moisture reading.

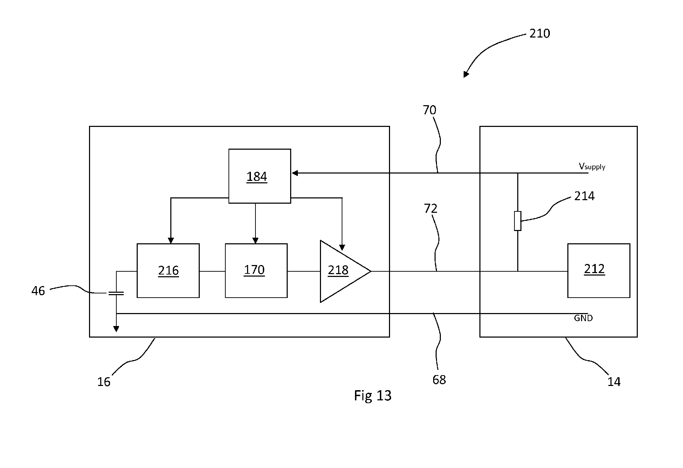

Figure 13 shows a schematic diagram for a moisture sensor 16 and control device 14 according to an embodiment, generally designated 210, for use with the irrigation apparatus 10, 100, 118, 120, 130, 140 of the above embodiments. In Figure 13 like features to the arrangements of Figure 1 - 12 are shown with like reference numerals. In Figure 13 the moisture sensor 16 is coupled to the control device 14 via the three wires 68, 70, 72 as shown in Figures 2 and 12. In Figure 13 the control device 14 has a microcontroller 212, also known as the processor described in relation to Figure 1. In Figure 13 a load resistor 214 is provided in the control device 14 between the positive supply wire 70 and the signal wire 72. The load resistor 214 may be for example 500 Ohms, and provides the advantage that the current through the wire 72 transmits the frequency signal from the moisture sensor 16. This reduces the effects of electrical interference upon accuracy because the communication of the moisture reading from the moisture sensor 16 is current-driven rather than voltage-driven, which provides a reduced sensitivity to electrical interference. This means that the signal voltage at the microcontroller 212 is independent of the resistance of the wire 72, which may vary depending on the length of the wire 72 between the moisture sensor 16 and the control device 14. If the communication was voltage-driven there would be a greater drop in voltage if the wire 72 was longer. In an alternative arrangement the control device 14 may be the master controller 12.

Also shown in Figure 13 is the moisture sensor 16 which comprises the voltage regulator circuit 184, the microcontroller 170, a sigma-delta modulator 216 and a buffer amplifier 218. The voltage regulator circuit 184 provides a stable power supply to the microcontroller 170, the sigma-delta modulator 216 and the buffer amplifier 218. Each moisture sensor 16 of the irrigation apparatus 10, 100, 118, 120, 130, 140 may include a respective buffer amplifier 218 to reduce the effect upon measurement of the length of the connecting wires 68, 70, 72 between each moisture sensor 16 and its associated control device 14 or master controller 12. The buffer amplifier 218 may assist with isolating the measurement part of the circuit diagrams 150, 180, from the communication part of the circuit diagrams 150, 180 which communicates the measurement over the connecting cable to the control device 14 or master controller 12. Hence variations in the length of the connecting wires 68, 70, 72 between the moisture sensor 16 and the control device 14 or the master controller 12 may have a reduced effect on the accuracy of the moisture level measurement. In one arrangement the buffer amplifier 218 is provided in the microcontroller 170.

Also shown in Figure 13 is the sigma-delta modulator 216 which may be used with the circuit diagram 150 shown in Figure 11. In Figure 13 the sigma-delta modulation technique may be used to reduce the effects of electrical interference upon measurement accuracy. Such a sigma-delta modulation technique is a known analogue-to-digital conversion that permits interference to be averaged over time so that the associated detrimental effect on measurement of the moisture content is reduced. Over the measurement period, the capacitance being measured by the sensing capacitor 46 will remain constant whilst the interference may vary so that when the signal is integrated over time by the microcontroller 170 or the data processing module 154 the ratio of the measured parameter compared to the interfering signal, i.e. signal-to-noise ratio, will be large.

The above arrangement shown in Figures 11 - 13 permit multiple moisture sensors 16 to be connected to a single cable which leads to the master controller 12 or the controller device 14. This is achieved using the connector block 209 whereby each moisture sensor 16 has a respective connector block 209 and the single cable has a plurality of connector blocks. It will be appreciated that the single cable comprises the three wires 68, 70, 72. Each moisture sensor 16 is connected in parallel to the single cable. This arrangement also allows for multiple sensors 16 to share and be connected to the same single cable using the respective connectors 209.

It will be appreciated that the oscillator circuit 48 may be configured so that it fits onto the arrangement of the moisture sensor 80, 90 shown in Figures 3 and 4. In the above embodiments the accuracy of the moisture reading provided by the sensing capacitor 46 is dependent on the repeatability and accuracy of the oscillator circuit 48. Since the oscillator circuit 48 is highly tolerant to climatic temperature and pressure changes due to its design, each moisture sensor 16 in the irrigation apparatus 10, 100, 118, 120, 130, 140 provides a very similar moisture reading to other sensing capacitors in the irrigation apparatus 10, 100, 118, 120, 130, 140. Furthermore, when relatively long wires are used to connect each moisture sensor 16 to the controller device 14 or the master controller 12, the circuits 150, 180, 210 may be less prone to electrical interference, for example due to radio waves or objects passing nearby such as trains or cars.

It will also be appreciated that the circuits 150, 180, 210 shown in Figures 11 - 13 can be used with the irrigation apparatus 10, 100, 118, 120, 130, 140 of the above embodiments to control the water supply 26 to the medium in which the moisture sensor 16 is located.

It will be appreciated that the circuit diagrams 150, 180 measure an analogue capacitance, and the tolerances within the components of the analogue circuit may cause variations between each physical device. Providing the microcontroller 170 with the programmable memory allows these variations to be accommodated as part of the production test procedure. The memory has a calibration table, such as a calibration multiplier and offset, to correct each moisture device 16. Furthermore, the circuit diagrams 150, 180, 210 operate to provide a signal which has a variable frequency as an indication of moisture content of the medium in which the moistures sensor 16 is located. Using frequency as the defining output parameter for moisture content has the advantage that any signal attenuation or variation due to the connecting cables will not affect this parameter.

The above described irrigation apparatus 10, 100, 118, 120, 130, 140 is capable of finely controlling the amount of water in the soil below the ground surface 30. The moisture sensor 16 may be arranged to report an analogue or a digital value of water content in the soil to the master controller 12 or the control device 14. A digital signal may provide the advantage of being less vulnerable to interference. The master controller 12 or the control device 14 then compares the value with a predetermined threshold value that has been programmed into the master controller 12 or the control device 14 by a user. The master controller 12 or the control device 14 then makes the decision to water or stop watering by initiating activation of the control valve 18 depending on whether the water content in the soil is below or above the predetermined threshold value. Such activation may be performed by simply providing power to the control valve 18 as per the irrigation apparatus 130, 140 of Figures 9 and 10, or by sending a command using a signal superimposed over the wires used to provide power to the control valve 18 as per the irrigation apparatus 10, 100, 118, 120 of Figures 1, 6, 7 and 8. In general the control valve 18 is operated on a cyclical basis, for example, once or twice per day for a period of one or more minutes. The period of time for which the control valve 18 remains open in any one cycle can be readily changed by the master controller 12 or the control device 14 depending on the reading obtained from the moisture sensor 16. Furthermore, the watering time may be adjusted depending on the soil condition just prior to watering. Other factors such as ambient temperature and wind speed may also be taken into account when adjusting the length of the watering time.

In one embodiment the irrigation apparatus 10, 100, 118, 120, 130, 140 uses a nominal 12V Direct Current provided by a power supply via a single wire pair which is laid around an area to be irrigated. A plurality of control devices 14 to be used in the irrigation apparatus 10, 100, 118, 120, 130, 140 are connected in parallel to the wire pair which is used to supply electrical power for all of the control devices 14. Connection of the control devices 14 to the wire pair may be made using a detachable connector such as a plug and socket connector. In turn the control devices 14 supply electrical power for the moisture sensors 16 and the control valves 18. In addition the same wire pair may be used for communication purposes between the control devices 14 and the master controller 12.

It will be appreciated that due to the feedback arrangement of the irrigation apparatus 10, 100, 118, 120, 130, 140 it is possible to compensate for a pressure drop along the delivery pipe 28. For example, if the water pressure within the delivery pipe 28 is significantly lower at the periphery of the area to be irrigated, the feedback arrangement of the irrigation apparatus 10 compensates by extending the watering time in that area.

In one embodiment the irrigation apparatus 10, 100, 118, 120 is able to perform a leak test to determine if the delivery pipe 28 or the control valve 18 is leaking. The leak test is performed using a second valve device 37 which has a pressure transducer to measure pressure in the delivery pipe 28 as shown in Figure 1. The master controller 12 is in communication with the second valve device 37 via the wires 20, 35. If there are many control devices 14 in the irrigation apparatus 10, the second valve device 37 is coupled to the last control device 14 associated with the delivery pipe 28, and the valve device 18 is coupled to the first control device 14 associate with the delivery pipe 28. To perform the leak test the master controller 12 is also required to know the addresses of control device 14 and the control valve 18 coupled to the second valve device 37. To perform the leak test the delivery pipe 28 is pressurised by closing the second valve device 37 and opening the control valve 18. The control valve 18 is then closed so that the delivery pipe 28 remains pressurised with water. The pressure in the supply pipe 28 is then monitored over a period of time with the pressure transducer. In one arrangement the pressure is measured every 10 seconds. If the pressure in the delivery pipe 28 reduces over time this is an indication that there is a leak in the delivery pipe 28, the second valve device 37, or the control valve 18. If one of the moisture sensors 16 measures a moisture content for the soil that is consistent very wet or above the predetermined threshold value, this may also indicate that there is a leak. Furthermore, if one of the moisture sensors 16 measures a moisture content for the soil that is consistently very dry or below the predetermined threshold value, this may indicate a problem with the control valve 18 failing to open. In either situation a faulty control valve 18 can be verified by performing a leak test by pressurising the delivery pipe 28 and operating the suspected faulty control valve 18 to check if the pressure reduces over time.

In the above embodiments the master controller 12 may or may not be included in the irrigation apparatus 10, 100, 118, 120, 130, 140. Whereas the master controller 12 may provide certain advantages, such as providing a central location for overall operation, it may be omitted such that the irrigation apparatus 10, 100, 118, 120, 130, 140 has a more basic mode of operation. In certain situations the basic mode of operation may be more advantageous. When the master controller 12 is omitted the one or more control devices 14 make the decision to operate the one or more control valves 18. In this arrangement the processor of the control device 14 performs the management and control functions of the irrigation apparatus 10, 100, 118, 120, 130, 140. In the case of more than one control device 14 the processors of each control device 14 are operable to communicate with one another to perform the operation and control functions of the irrigation apparatus 10, 100, 118, 120, 130, 140.

In one arrangement of the above embodiments 10, 100, 118, 120, 130, 140 the delivery pipe 28 used in any of the above embodiments is connected to the control valve 18 at one end, and is closed at the other end. The series of apertures 32 along the length of the delivery pipe 28 permits water to be delivered to the area being irrigated. The delivery pipe 28 may have a relatively large bore, such as a diameter of 32mm. This has the advantage that the delivery pipe 28 operates as a reservoir of water under pressure. Such a reservoir has the advantage that the apertures 32 which are further away from the control valve 18 do not emit significantly less water than apertures 32 which are closer to the control valve 18. It will be appreciated that the delivery pipe 28 may be a hose pipe. If the hose pipe has a relatively small diameter it may not act as a reservoir. If the diameter of the hose pipe is too large the delivery pipe 28 is awkward to handle. Having a diameter of 32mm, and between 25 - 40 mm, is a convenient size to handle whilst also providing a sufficient reservoir. With a smaller installation of the irrigation apparatus 10, 100, 118, 120, 130, 140 the hose pipe may have a diameter of between 12 - 16mm. Accordingly, with the irrigation apparatus 10, 100, 118, 120, 130, 140 the hose pipe may have a diameter of between 12 - 50mm.

It will be appreciated that the above described embodiments may find application for irrigation in gardens, or farming applications such as in glasshouses or fields for growing crops, fruit or other plants.

In the above described embodiments it will be appreciated that the electrodes 42, 44 of the sensing capacitor 46 are insulated from each other and from the medium in which they are located. Accordingly the electrodes 42, 44 may alternatively be termed insulated electrodes.

## Claims

1. A moisture sensor for an irrigation apparatus, the moisture sensor being operable to monitor a moisture content for a medium in which it is located to provide a control signal for operating the irrigation apparatus for controlling a water supply to the medium, the moisture sensor comprising a sensing capacitor, wherein the moisture sensor comprises an oscillator circuit coupled to the sensing capacitor.

2. A moisture sensor according to claim 1, wherein the oscillator circuit comprises an operational amplifier.

3. A moisture sensor according to claim 2, wherein the operational amplifier is operable to charge and discharge the sensing capacitor to provide an output signal from the oscillator circuit having a frequency being dependent on a moisture content of a medium in which the moisture sensor is located.

4. A moisture sensor according to claim 3, wherein a microcontroller is operable to receive the output signal to determine the frequency thereof.

5. A moisture sensor according to any preceding claim, wherein the oscillator circuit comprises a first semiconductor switch, wherein the sensing capacitor is coupled to a modulating capacitor which is chargeable via the first semiconductor switch.

6. A moisture sensor according to claim 5, wherein the oscillator circuit comprises a second semiconductor switch which is operable to alternatively couple the sensing capacitor to a resistor and the modulating capacitor.

7. A moisture sensor according to claim 5 or 6, when dependent on claim 2, wherein the modulating capacitor is coupled to the operational amplifier, such that when a high voltage threshold of the modulating capacitor is reached the operational amplifier has a low voltage output, and when a low voltage threshold of the modulating capacitor is reached the operational amplifier has a high voltage output.

8. A moisture sensor according to claim 7, or claims 2 - 4, wherein the operational amplifier is coupled to a pulse counter which is operable to register a count according to the voltage output from the operational amplifier.

9. A moisture sensor according to claim 8, wherein the pulse counter comprises a microcontroller which is operable to determine the frequency of counts.

10. A moisture sensor according to claim 9, wherein the microcontroller is programmable to determine the number of counts over a preset time period to determine the frequency of counts, the preset time period being adjustable.

11. A moisture sensor according to claim 9 or 10, wherein the microcontroller has a programmable memory to calibrate the oscillator circuit and/or the sensing capacitor.

12. A moisture sensor according to any preceding claim, wherein a power source for the oscillator circuit comprises a voltage regulator.

13. A moisture sensor according to any preceding claim, and further including a buffer amplifier.

14. An irrigation apparatus including a moisture sensor according to any of claims 1 - 13.

15. A method of using a moisture sensor in an irrigation apparatus, the moisture sensor comprising a sensing capacitor, the method including using the sensing capacitor to measure a moisture content for a medium in which the moisture sensor is located to provide a control signal, and operating a water supply to the medium in response to the control signal, wherein the moisture sensor comprises an oscillator circuit coupled to the sensing capacitor, the method including charging and discharging the sensing capacitor using the oscillator circuit to provide an output signal from the oscillator circuit that has a frequency which is dependent on a moisture content of the medium.
